# EUROPEAN PATENT APPLICATION

(11) **EP 2 740 426 A1**
(43) Date of publication of application: **11.06.2014**
(21) Application number: 13192806.1
(22) Date of filing: 14.11.2013
(51) Int. Cl.: A61B 17/84, A61B 19/00, A61M 25/09

(54) **Graduated guide pin for use in medical treatment**

(30) Priority: 06.12.2012 JP 2012267374
(71) Applicant: Tama Medical Co Ltd, Hussa-shi, Tokyo (JP)
(72) Inventor: Machida, Eiichi, Hussa-shi, Tokyo (JP)
(74) Representative: Hartley, Andrew Philip

(57) **Abstract**

A graduated guide pin for selecting a screw for connecting a fractured bone is formed by a pinlike main body 11 of a radiotransparent metal and ring-like members 12 of a radioopaque metal. The ring-like members are arranged on an outer surface of the main body such that successive members are separated from each other by a distance equal to that of the ring-like members. On an X-ray image, by counting the number of dark portions corresponding to the ring-like members, a length of the fractured bone is measured, and a screw 20 having a suitable length is selected.

## Description

### Background of the Invention

### Field of the Invention

The present invention relates to a graduated guide pin for use in a medical treatment, and more particularly relates to a graduated guide pin to be used in an osteosynthesis for connecting a fractured bone by means of a screw, in which the guide pin is inserted into the fractured bone for determining a most suitable length of a screw.

### Related Art Statements

When a fracture of a bone occurs in accidents or playing sports, a surgical operation called osteosynthesis is conducted for connecting a fractured bone by using a medical screw called a cannulated screw. Upon performing this surgical operation, at first it is required to fasten a cannulated screw having a best suitable length among various kinds of screws having different lengths.

A bone is consisting of an outer cortical bone made of hard tissues and an inner cancellous bone made of soft spongiform tissues. If use is made of a cannulated screw having a length which is longer than a desired length, a tip of the screw might extrude beyond a cortical bone on an opposite side and might injure subcutaneous tissues. On the contrary, if a cannulated screw is too short, a tip of the screw might not reach the hard cortical bone and might stay within the soft cancellous bone. Therefore, the screw could not be fixed firmly and the connection of fractured bone might be failed.

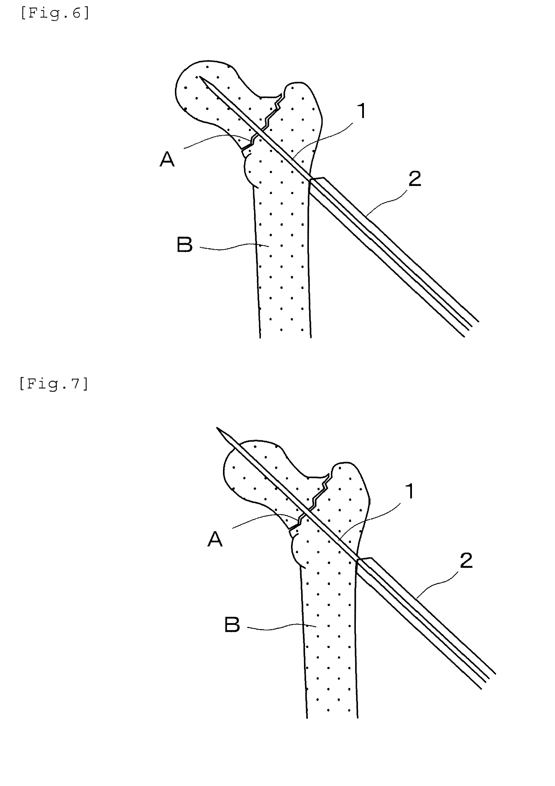

In the following Japanese Patent document 1, there is disclosed an operation for connecting a fractured bone. At first, a guide pin 1 is inserted into a fractured bone piece B toward a fracture site A in such a direction that the guide pin 1 extends substantially perpendicularly to a fracture line as illustrated in Fig. 6. While monitoring an X-ray image obtained by a radioscopic screen or X-ray film, it is confirmed that the guide pin 1 has been inserted into a desired position. Then, a length of a portion of the guide pin 1 inserted into the bone is measured by means of a guide pin gauge 2. Since a whole length of the guide pin 1 is known, a length of the guide pin portion inserted into the bone can be estimated by measuring a portion of the guide pin 1 which is not inserted into the bone by means of the guide pin gauge 2.

A cannulated screw having a most suitable length is selected in accordance with the measured length of the guide pin portion inserted into the fractured bone, and the guide pin 1 is inserted into a central hole formed in the selected screw until a front end of the screw is brought into contact with the bone piece B. After that, the screw is inserted into the fractured bone by means of a driver until a tip of the screw is inserted into a cortical bone of the fractured bone piece B. In this manner, the fractured bone piece B is firmly fixed and is connected to the fractured bone A. After several months from the operation, the fractured bone piece B is completely connected to the bone A, and the screw is removed from the thus connected bone.

### Prior Art Publications

[Patent Publication 1] Japanese Patent Publication Kokai-Hei 9-220235

### Summary of the Invention

### Problems to be Solved by the Invention

When the guide pin 1 is inserted into the fractured bone A, B, the guide pin penetrates a front side hard cortical bone and is further inserted into the soft cancellous bone. Then, a cone drill provided at a tip of the guide pin 1 is inserted into subcutaneous tissues through a remote side hard cortical bone. Since the cortical bone is hard, the guide pin 1 must be inserted with a rather large force, and therefore the tip of the guide pin 1 might protrude from the remote side cortical bone excessively as depicted in Fig. 7.

If the guide pin 1 protrudes excessively, a length of a portion of the guide pin inserted into the fractured bone measured with the guide pin gauge 2 is longer than a desired value. In this manner, a length of a portion of the inserted guide pin 1 inserted into the fractured bone could not be measured correctly, and therefore a cannulated screw having a most desirable length could not be selected.

It would be possible to correct a length of the inserted guide pin 1 measured by the guide pin gauge 2 by estimating a length of a portion of the guide pin 1 excessively protruded from the remote side cortical bone. However, usually the X-ray image is taken from a direction which is inclined with respect to a direction perpendicular to the guide pin 1. Therefore, a length measured from the X-ray image is not accurate. For instance, a portion situating remote from an X-ray tube is seen with an enlarged size. In this manner, a length of a portion of the guide pin excessively protruding from the remote side cortical bone could not be estimated correctly in units of millimeter.

Therefore, in conventional operations, an operator draws the guide pin 1 slightly, and then an X-ray image is obtained again and it is necessary to confirm that the guide pin 1 has been inserted into a desired position. In some case, the guide pin 1 might be drawn excessively. In such a case, an X-ray image has to be formed again after slightly inserting the guide pin 1.

When use is made of the guide pin gauge 2, after cutting subcutaneous tissues, cortical substances and bone and the guide pin 1 is inserted into a central hole formed in the gauge 2, the gauge 2 is moved toward the fractured bone until the tip of the gauge is brought into contact with the surface of the fractured bone. This treatment is rather cumbersome. Moreover, it is not easy to read accurately the scales formed on the guide pin gauge 2.

The present invention has for its object to remove the above mentioned problems and to provide a novel and useful graduated guide pin for determining a suitable length of a screw in connecting a fractured bone. Means for Solving the Problems

According to the invention, a graduated guide pin for use in surgical operation comprises a pin-like main body made of a radiotransparent metal, said pin-like main body having a cone-shaped drill formed at a front end thereof; and a plurality of ring-like members made of a radioopaque metal, said ring-like members being successively arranged on an outer surface of the pin-like main body equidistantly along a longitudinal direction with a given length, whereby the gradated guide pin has a substantially smooth outer surface.

According to another aspect of the invention, a graduated guide pin for use in surgical operation comprises a pin-like main body made of one of a radiotransparent metal and a radioopaque metal and having a cone-shaped drill formed at a front end thereof, said main body having a plurality of ring-like recesses formed in an outer surface and being successively arranged equidistantly along a longitudinal direction with a given distance; and a plurality of ring-like members made of the other one of the radiotransparent metal and a radioopaque metal or, each of said ring-like members being embedded within respective one of said ring-shaped recesses formed in the outer surface of the main body such that the guide pin has a smooth outer surface.

### Merits of the Invention

In the graduated guide pin for use in surgical operation according to the invention, it is possible to measure a length of a portion of the guide pin inserted into a fractured bone without using a guide pin gauge and a fractured bone connecting screw having a desired length can be selected accurately and easily. Moreover, even if the guide pin has inserted into the bone excessively, a screw having a desired length can be determined without monitoring radioscopic image or X-ray film image repeatedly.

### Brief Description of the Drawings

Fig. 1 is a side view showing a first embodiment of the graduated guide pin according to the invention;
Fig. 2 is an explanatory view of an X-ray image obtained after inserting the guide pin into a fractured bone;
Fig. 3 is an explanatory view of an X-ray image showing a condition in which a fractured bone is fixed by a cannulated screw;
Fig. 4 is an explanatory view depicting a condition in which a guide pin is used for fixing a metal plate onto a fractured bone;
Fig. 5 is a cross sectional view illustrating a second embodiment of the graduated guide pin according to the invention;
Fig. 6 is an explanatory view showing a manner of measuring a length of a portion of a guide pin inserted into a fractured bone in a conventional method; and
Fig. 7 is an explanatory view showing a condition in which a length of a portion of a guide pin inserted excessively into a fractured bone in a conventional method.

### Description of the Preferred Embodiments

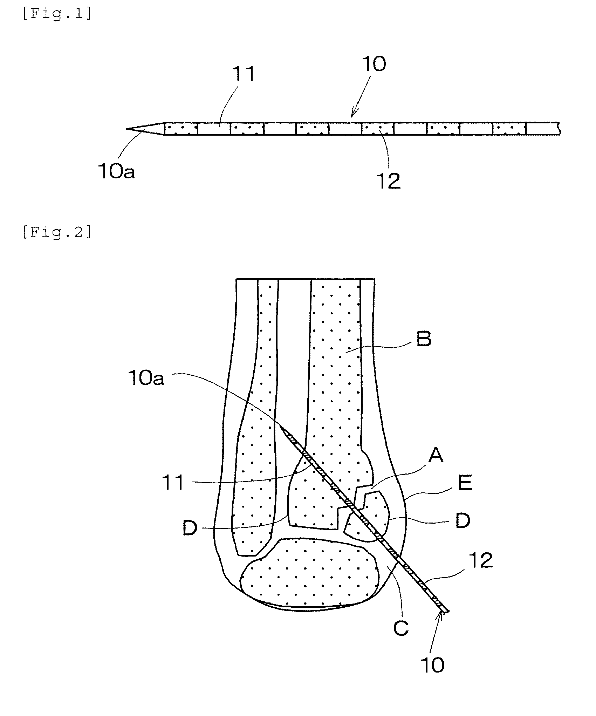

Now the present invention will be explained in detail with reference to embodiments shown in Figs. 1-5.

### Embodiment 1

Fig. 1 is a side view showing a first embodiment of the graduated guide pin according to the invention. A guide pin 10 comprises a pin-like main body 11 having a diameter of about 2-4 mm and a length of about 30 cm. One end of the guide pin 10 is formed into a sharp cone-shaped drill 10a such that the guide pin can be easily inserted into a fractured bone. The main body 11 is made of a radiotransparent metal. The guide pin 10 further comprises a plurality of ring-like members 12 made of a radioopaque metal and arranged on an outer surface of the main body 11. The ring-like members 12 have a given axial length of, for example 5 mm and are successively arranged around the pin-like main body 11 along a longitudinal direction with interposing a space whose axial length is equal to the axial length of said ring-like members 12. In this manner, a combination of the main body 11 made of a radiotransparent metal and the ring-like members 12 made of a radioopaque metal constitutes a graduation or scale. The ring-like members 12 may be applied on the main body 11 by various methods such as plating, sputtering, coating, welding and sticking. In this case, it should be noted that since the ring-like members 12 have a very small thickness, an outer surface of the guide pin 10 is substantially smooth so that the guide pin 10 can be easily and smoothly inserted into and drawn out of a fractured bone.

The guide pin 10 can be simply inserted into soft subcutaneous tissues by pushing, but when the guide pin 10 is to be inserted into a hard bone, the guide pin 10 is rotated such that an opening is formed in the bone by means of the cone-shaped drill 10a.

A plurality of guide pins 10 having different total lengths, diameters and lengths of ring-like members are prepared, and an operator selects a suitable guide pin 10 in accordance with a fractured bone. In this manner, a most suitable screw can be selected correctly. In Fig. 1, the ring-like members 12 are denoted to have a different color from the main body 11 for the sake of clarity, but according to the invention, the ring-like members 12 may have the same color as the main body 11. That is to say, it is not necessary to distinguish the guide pins 10 by the outer appearance.

In this embodiment, the main body 11 is made of a metal having a high transmissivity for radiation such as titanium and titanium alloy, i.e. a metal having a smaller atomic number in the periodic table. In the present invention, such a metal is called a radiotransparent metal. The ring-like members 12 are made of a metal having a low transmissivity for radiation such as platinum, iridium, tungsten, tantalum, gold and alloys of these metals, i.e. a metal having a larger atomic number in the periodic table. In the present invention, such a metal is termed as a radioopaque metal.

Fig. 2 is an X-ray image displayed on a radioscopic screen; in which a bone B is fractured at a fracture site A. Upon the osteosynthesis treatment for a fractured bone B, at first the fractured bone B is moved under anesthesia such that the fractured bone is returned or reformed into an original position as far as possible. Then, a remote end of the guide pin 10 is fixed to a chuck of a motor drill, and the guide pin 10 is inserted into the fractured bone B via a skin E and subcutaneous tissues C. In this case, the guide pin 10 is inserted such that the guide pin extends in a direction substantially perpendicular to a fracture line at the fracture site A. When the cone drill tip 10a of the guide pin 10 is brought into contact with a hard cortical bone D of a fractured bone piece, the motor drill is actuated to rotate the guide pin 10. Then, the guide pin 10 is inserted into the fractured bone B through the fractured bone piece and fractured site A. Further, the guide pin 10 extrudes out of the fractured bone B through a hard cortical bone D on a remote side into soft subcutaneous tissues.

As explained above, the ring-like members 12 have a length of 5mm and are separated from each other by the same length of 5mm. Therefore, on the X-ray image, the bright and dark portions appear alternately and each of these portions has a length corresponding to 5 mm. It should be noted that a length of about 1 mm can be estimated by monitoring the image of the guide pin 10. In this manner, a length of a portion of the guide pin inserted into the fractured bone B can be measured accurately in the units of mm.

In the condition shown in Fig. 2, the guide pin 10 protrudes excessively from the cortical bone of the fractured bone B into the subcutaneous tissues C. The excessively protruding portion has a length longer than 10 mm. Even in such a case, according to the invention, it is possible to measure a length of a portion of the guide pin 10 inserted into the fractured bone B including the fracture site A by counting the number of bright and dark portions. In this case, this length is estimated as 53 mm. In this manner, a most suitable length of a cannulated screw 20 can be determined from a single X-ray image on the radioscopic screen or film. As stated above, according to the invention, even if the X-ray image is taken from an inclined direction, a length of the fractured bone B including the fracture site A can be measured by counting the number of bright and dark portions within the fractured bone B.

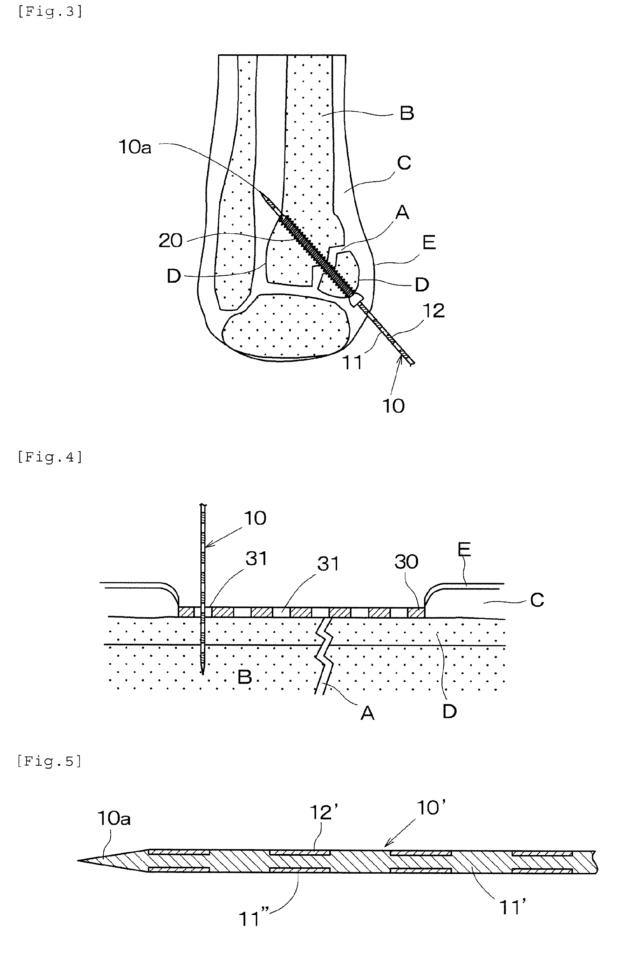

As illustrated in Fig. 3, after the measurement, a screw having a most suitable length is selected from a number of screws having different lengths. In the present case, a screw 20 having a length of 55 mm is selected. Then the selected screw 20 is screwed into the fractured bone B from the front side cortical bone D. In this manner, the fractured bone piece can be fixed to the remaining portion of the fractured bone B. In the present embodiment, the screw 20 is of the cannulated type, the guide pin 10 is inserted into a central hole formed in the screw such that the screw 20 is guided by the guide pin 10. After fixing the screw 20, the guide pin 10 is drawn out of the fractured bone B, and the injured skin E is put in a suture. According to the invention, a solid type screw having no central hole may be also used. In such a case, after the measurement, the guide pin 10 is removed from the fractured bone B.

In Figs. 2 and 3, the osteosynthesis treatment is carried out by inserting the screw such that the screw penetrates through the fractured bone B. The guide pin 10 according to the invention may be used in the osteosynthesis treatment using a splint plate 30 shown in Fig. 4. In this case, the guide pin 10 is inserted into the fractured bone B through one of a plurality of openings 31 formed in the splint plate 30. By monitoring an X-ray image, a most suitable length of a screw is selected. After fixing the thus selected screw, the guide pin 10 is removed. This operation is repeated for the remaining openings 31 formed in the splint plate 30. In this manner, the fractured bone B including the fracture site A can be fixed by means of the splint plate 30.

In the present embodiment, each of the ring-like members 12 has a length which is equal to a length of the space separating adjacent to ring-like members 12. However, according to the invention, a length of the ring-like members 12 may be smaller than a length of the space separating ring-like members along the longitudinal direction of the base material 11 so long as the ring-like members and the space are alternately arranged with a given distance. Therefore, the ring-like members 12 may have a given axial length of, for example 5 mm and successively arranged around the pin-like main body 11 along a longitudinal direction with interposing a space whose axial length is 5 mm. In this case, on the X-ray image, the dark portions of the ring-like members 12 are displayed as scale lines, and therefore by counting the number of scale lines within the fractured bone B, it is possible to measure a length of the fractured bone B including the fracture site A.

### [Embodiment 2]

In the graduated guide pin 10 of the first embodiment, the ring-like members 12 might be separated by rubbing during repeated use. In a graduated guide pin 10' of a second embodiment of the invention, ring-like recesses 11" are formed in an outer surface of a pin-like main body 11' as illustrated in Fig. 5. The ring-like recesses 11" have a given axial length and are successively arranged around the pin-like main body 11' along a longitudinal direction with interposing a space whose axial length is equal to the axial length of said ring-like recesses 11". A plurality of ring-like members 12' made of a radioopaque metal are provided within the ring-like recesses 11" such that an outer surface of the thus formed guide pin 10' is highly smooth. The ring-like members 12' may be provided within the ring-like recesses 11" by various methods such as attaching, fitting and embedding.

In this guide pin 10', the ring-shaped members 12' could not be separated by rubbing. If use is made a guide pin in which the ring-like recesses 11" are not filled with the ring-like members 12', it would be possible to measure a length of a portion of the guide pin inserted into a fractured bone by counting recesses displayed on an X-ray image. However, such a guide pin has protrusions and depressions formed in an outer surface, the subcutaneous tissues C might be damaged during the inserting and removing operation of the guided pin. Moreover, when the guide pin is inserted into the fractured bone including a fracture site A, the guide pin might be broken or bent, because a mechanical strength of the guide pin is not sufficiently large. Furthermore, when the cannulated screw 20 having the central hole as shown in Fig. 3 is used, the screw could not be moved smoothly along the guide pin. Contrary to this, since the guide pin 10' of the present embodiment has not any protrusions and depressions formed in the outer surface of the guide pin 10', the above-mentioned drawbacks do not occur.

According to the invention, a length of the ring-like recesses 11" may be smaller or larger than the space separating ring-like members along the longitudinal direction of the base material 11 so long as the ring-like members and the spaces are alternately arranged with given distances. In general, a length of the ring-like members 11" may be determined such that the ring-like members 12' can be provided easily within the ring-like recesses 11". Moreover, according to the invention, it is not always necessary that the ring-like recesses 11" and ring-like members 12' have a completely continuous ring shape. That is to say, the ring-like recesses 11" and ring-like members 12' may have any shape so long as the number of the ring-like members 12' can be counted on the X-ray image and the outer surface of the guide pin 10' is smooth and does not have protrusions and depressions. In such a case, metal chips are placed within the ring-like recesses 11" and are fused to form the ring-like members 12' within the ring-like recesses 11".

In this case, a dark portion of the ring-like member 12' displayed on the X-ray image is not a unit body, but is divided into two or more than two parts, however, the number of the ring-like members 12' can be counted without difficulty. A length and a thickness of the ring-like recesses 11" and ring-like member 12' may be suitably determined such that dark portions of the ring-like members 12' on the X-ray image can be clearly distinguished from the bright portions corresponding to the pin-like main body 11'.

In the second embodiment, the pin-like main body 11' is made of a radiotransparent metal and the ring-like members 12' are made of a radioopaque metal, however according to the invention, the pin-like main body 11' may be made of a radioopaque metal and the ring-like members 12' may be made of a radiotransparent metal. In such a case, a length of a portion of the guide pin 10' may be measured by counting the number of bright portions of the ring-like members 12' displayed on the X-ray image.

The graduated guide pins of the above explained first and second embodiments may be used in a dental treatment such as cutting a nerves in a decayed tooth. In such a case, the guide pin is inserted to a root canal of the decayed tooth and a distance to the nerves can be measured on the X-ray image.

### [Description of the Reference Numerals]

10, 10' denote a graduated guide pin, 11, 11' a main body, 11" a recess, 12, 12' a ring-like member, 20 a screw and 30 represents a splint plate.

## Claims

1. A graduated guide pin for use in surgical operation comprising a pin-like main body made of a radiotransparent metal, said pin-like main body having a cone-shaped drill formed at a front end thereof; and a plurality of ring-like members made of a radioopaque metal, said ring-like members being successively arranged on an outer surface of the pin-like main body equidistantly along a longitudinal direction with a given length, whereby the graduated guide pin has a substantially smooth outer surface.

2. The graduated guide pin for use in surgical operation according to claim 1, the ring-like members have a given axial length and are successively arranged around the pin-like main body along a longitudinal direction with interposing a space whose axial length is equal to the axial length of said ring-like members.

3. A graduated guide pin for use in surgical operation comprising a pin-like main body made of one of a radiotransparent metal and a radioopaque metal and having a cone-shaped drill formed at a front end thereof, said main body having a plurality of ring-like recesses formed in an outer surface and being successively arranged equidistantly along a longitudinal direction with a given distance; and a plurality of ring-like members made of the other one of the radiotransparent metal and a radioopaque metal or, each of said ring-like members being embedded within respective one of said ring-shaped recesses formed in the outer surface of the main body such that the guide pin has a smooth outer surface.

4. The graduated guide pin according to claim 3, wherein ring-like recesses having a given axial length are formed in an outer surface of a pin-like main body along a longitudinal direction with interposing a given space whose axial length is equal to the axial length of said ring-like recesses.

5. The graduated guide pin according to any one of claims 1-4, wherein said radiotransparent metal is a metal selected from a group consisting of titanium and titanium alloy.

6. The graduated guide pin according to any one of claims 1-5, wherein said radioopaque metal is a metal selected from a group consisting of platinum, iridium, tungsten, tantalum, gold and alloys of these metals.

7. The graduated guide pin according to any one of claims 1-4, wherein the guide pin performs a roll of guide such that the guide pin is inserted into an opening of a medical screw.
